# EUROPEAN PATENT SPECIFICATION

(11) **EP 4 166 185 B1**
(45) Date of publication and mention of the grant of the patent: **02.12.2026**
(21) Application number: 22830358.2
(22) Date of filing: 06.07.2022
(51) Int. Cl.: A61M 21/00

(54) **METHOD AND APPARATUS FOR GENERATING SLEEP-AID MUSIC**
VERFAHREN UND VORRICHTUNG ZUR ERZEUGUNG VON MUSIK ZUR UNTERSTÜTZUNG DES SCHLAFES
PROCÉDÉ ET APPAREIL DE GÉNÉRATION DE MUSIQUE D'AIDE AU SOMMEIL

(30) Priority: 25.08.2021 CN 202110984332
(43) Date of publication of application: 19.04.2023
(73) Proprietor: Anhui Huami Health Technology Co., Ltd., Hefei, Anhui 243000 (CN)
(72) Inventor: ZHANG, Cong, Hefei, Anhui 243000 (CN); MENG, Zi, Hefei, Anhui 243000 (CN); ZHU, Guokang, Hefei, Anhui 243000 (CN); YU, Yi, Hefei, Anhui 243000 (CN)
(74) Representative: Angerhausen, Christoph
(86) International application number: PCT/CN2022/104139
(87) International publication number: WO 2023/024717

(56) References cited:
- EP-A1- 3 669 922
- CN-A- 103 768 698
- CN-A- 105 374 347
- CN-A- 106 652 984
- CN-A- 111 821 556
- CN-A- 113 032 615
- GB-A- 2 475 096
- US-A1- 2017 140 743
- DAVID DANIEL ALBARRACIN MOLINA: "Adaptive music: Automated music composition and distribution", ARXIV.ORG, CORNELL UNIVERSITY LIBRARY, 201 OLIN LIBRARY CORNELL UNIVERSITY ITHACA, NY 14853, 25 July 2020 (2020-07-25), XP081737669
- WU YUE-BEI: "On the Technique of Spectral Composition Based on Algorithm", HUANGZHONG (JOURNAL OF WUHAN CONSERVATORY OF MUSIC,CHINA), no. 4, 25 October 2010 (2010-10-25), pages 3 - 10+203, XP009543801, ISSN: 1003-7721

## Description

### TECHNICAL FIELD

Embodiments of the present disclosure relate to the field of computer technologies, in particularly relate to methods, apparatuses, computer devices and storage medium for generating sleeping aid audio.

### BACKGROUND

Sleep can not only eliminate fatigue and create new vitality, but also improve immunity and disease resistance. Good immunity comes from good sleep. Music, as a multimedia carrier, is easy to be obtained and accepted, and has become primary means for sleeping aid. Music-based sleeping aid belongs to the category of psychological intervention, and aims to reduce stress and obtain physical and mental relaxation to achieve an effect of sleeping aid.

The sleeping aid audio used in related technologies is mostly created manually. However, the emotion expressed by this kind of sleeping aid audio is influenced by musicians' subjective ideas, and this kind of sleeping aid audio has a limited effect and application scope. Therefore, how to generate sleeping aid audio scientifically and effectively is an urgent problem to be solved.

DAVID DANIEL ALBARRACIN MOLINA: "Adaptive music: Automated music composition and distribution", ARXIV.ORG, CORNELL UNIVERSITY LIBRARY, 201 OLIN LIBRARY CORNELL UNIVERSITY ITHACA, NY 14853, 25 July 2020 XP081737669, discusses a new genetic algorithm consisting basically of (a) establishing the genetic operators; and (b) building a more comprehensive kind of fitness function, by grouping many of the rules that have been incorporated along the different stages of the compositional process. The purpose of implementing the GA is to open the range of musical parameters to produce the first population of compositions and then to refine the outcomes over generations with a biased evaluation function, which is intended to give place to more unexpected and creative solutions.

EP3669922A1 discloses a system and method for adapting the generation of an audio output based on a response of a subject's sleep parameters to the audio output. In particular, one or more rules used to generate the audio output are modified in response to how values of sleep parameters (i.e. parameters responsive to a sleep state of the subject) change in response to the audio output. The audio output can be iteratively modified to assess the impact of different audio outputs.

### SUMMARY

The present disclosure provides a method, an apparatus, a device and a storage medium for generating sleeping aid audio.

According to a first aspect of the present disclosure, a method for generating sleeping aid audio is provided, and includes:
obtaining a plurality of sleeping aid audio spectrums;
processing the plurality of sleeping aid audio spectrums with a genetic algorithm, to obtain a sleeping aid audio spectrum chain; and
generating the sleeping aid audio according to the sleeping aid audio spectrum chain.

In some embodiments, obtaining a plurality of sleeping aid audio spectrums includes:
obtaining m reference audio spectrums;
identifying each reference audio spectrum with an identification model generated by training, to determine a sleeping aid value of the each reference audio spectrum; and
determining n reference audio spectrums from the m reference audio spectrums as the plurality of sleeping aid audio spectrums, in which each of the n reference audio spectrum corresponds to a sleeping aid value greater than a first threshold, m is greater than n and n is a natural number greater than 1.

In some embodiments, processing the plurality of sleeping aid audio spectrums with the genetic algorithm, to obtain the sleeping aid audio spectrum chain includes:
processing the plurality of sleeping aid audio spectrums with the genetic algorithm based on the sleeping aid values corresponding to the plurality of sleeping aid audio spectrums, to obtain the sleeping aid audio spectrum chain.

In some embodiments, processing the plurality of sleeping aid audio spectrums with the genetic algorithm based on the sleeping aid values corresponding to the plurality of sleeping aid audio spectrums, to obtain the sleeping aid audio spectrum chain includes:
selecting one or more target sleeping aid audio spectrums to be processed from the plurality of sleeping aid audio spectrums based on the sleeping aid values corresponding to the plurality of sleeping aid audio spectrums;
processing the one or more target sleeping aid audio spectrums by performing at least one of a crossover operation and a mutation operation, to generate a plurality of first-generation sleeping aid audio spectrums;
selecting one or more target first-generation sleeping aid audio spectrums from the plurality of first-generation sleeping aid audio spectrums based on the sleeping aid values corresponding to the plurality of first-generation sleeping aid audio spectrums; and
preforming, repeatedly until a number of operations performed reaches a preset value, at least one of the crossover operation and the mutation operation based on the one or more target first-generation sleeping aid audio spectrums, and
determining the sleeping aid audio spectrum chain according to the one or more target sleeping aid audio spectrums and the generated respective generations of target sleeping aid audio spectrums.

In some embodiments, after the generating sleeping aid audio, the method further includes:
obtaining a sleep state of a user while playing the sleeping aid audio;
determining the sleeping aid value of the sleeping aid audio according to the sleep state; and
removing, in response to the sleeping aid value of the sleeping aid audio being less than a second threshold, a sleeping aid audio spectrum from which the sleeping aid audio is generated from the plurality of sleeping aid audio spectrums, to obtain a plurality of sleeping aid audio spectrums updated.

In some embodiments, obtaining the sleep state of the user includes:
obtaining a plurality of physiological parameters of the user collected by a wearable device, in which the plurality of physiological parameters include at least one of: a number of times of turn-overs, a heart rate, a blood pressure, a respiratory rate or a head motion frequency; and
determining the sleep state of the user according to the plurality of physiological parameters.

According to a second aspect of the present disclosure, an apparatus for generating sleeping aid audio is provided, and includes:
a first acquiring module, configured to obtain a plurality of sleeping aid audio spectrums;
a second acquiring module, configured to process the plurality of sleeping aid audio spectrums with a genetic algorithm, to obtain a sleeping aid audio spectrum chain; and
a first generating module, configured to generate the sleeping aid audio according to the sleeping aid audio spectrum chain.

**In** some embodiments, the first acquiring module is configured to:
obtain m reference audio spectrums;
identify each reference audio spectrum with an identification model generated by training, to determine a sleeping aid value of the each reference audio spectrum; and
determine n reference audio spectrums from the m reference audio spectrums as the plurality of sleeping aid audio spectrum, in which each of the n reference audio spectrum corresponds to a sleeping aid value greater than a first threshold, m is greater than n and n is a natural number greater than 1.

**In** some embodiments, the second acquiring module includes:
a processing unit, configured to process the plurality of sleeping aid audio spectrums with the genetic algorithm based on the sleeping aid values corresponding to the plurality of sleeping aid audio spectrums, to obtain the sleeping aid audio spectrum chain.

**In** some embodiments, the processing unit is configured to:
select one or more target sleeping aid audio spectrums to be processed from the plurality of sleeping aid audio spectrums based on the sleeping aid values corresponding to the plurality of sleeping aid audio spectrums;
process the one or more target sleeping aid audio spectrums by performing at least one of a crossover operation and a mutation operation, to generate a plurality of first-generation sleeping aid audio spectrums;
select one or more target first-generation sleeping aid audio spectrums from the plurality of first-generation sleeping aid audio spectrums based on the sleeping aid values corresponding to the plurality of first-generation sleeping aid audio spectrums; and
preform, repeatedly until a number of operations performed reaches a preset value, at least one of the crossover operation and the mutation operation based on the one or more target first-generation sleeping aid audio spectrums, and
determine the sleeping aid audio spectrum chain according to the one or more target sleeping aid audio spectrums and generated respective generations of target sleeping aid audio spectrums.

**In** some embodiments, the first generating module includes:
a first acquiring unit, configured to obtain a sleep state of a user while playing the sleeping aid audio;
a first generating unit, configured to determine the sleeping aid value of the sleeping aid audio according to the sleep state; and
a second generating unit, configured to remove, in response to the sleeping aid value of the sleeping aid audio being less than a second threshold, a sleeping aid audio spectrum from which the sleeping aid audio is generated from the plurality of sleeping aid audio spectrums, to obtain a plurality of sleeping aid audio spectrums updated.

**In** some embodiments, the first acquiring unit is configured to:
obtain a plurality of physiological parameters of the user collected by the wearable device, in which the plurality of physiological parameters include at least one of: a number of times of turn-overs, a heart rate, a blood pressure, a respiratory rate or a head motion frequency; and
determine the sleep state of the user according to the plurality of physiological parameters.

According to a third aspect of the disclosure, an electronic device is provided, and includes:
at least one processor; and
a memory connected by communication with at least one processor; in which
the memory stores instructions that may be executed by at least one processor, and the instructions are executed by at least one processor, so that at least one processor may execute the methods described in the embodiments in the above aspects.

According to a fourth aspect of the disclosure, a non-instantaneous computer readable storage medium that stores computer instructions is provided, and the computer instructions are executed by the computer to perform the method described in the embodiments of the above aspects.

According to a fifth aspect of the disclosure, a computer program product is provided, including a computer program, the computer program is executed by the processor to perform a method of any one of the embodiments of the above aspects.

It should be understood that the content described in this section is not intended to identify the key or important features of embodiments of the disclosure, nor intended to limit the scope of the disclosure. Other features of the disclosure will be easily understood from the following description.

**In** an embodiment of the present disclosure, a plurality of sleeping aid audio spectrums are obtained, and the plurality of sleeping aid audio spectrums are processed with a genetic algorithm, to obtain a sleeping aid audio spectrum chain, and the sleeping aid audio is generated according to the sleeping aid audio spectrum chain. Therefore, the sleeping aid audio spectrums may be used as genetic material to generate sleeping aid audio by using the genetic algorithm, which not only ensures an effect and reliability of sleeping aid audio, but also reduces a cost of sleeping aid audio.

Further, in an embodiment of the disclosure, a plurality of reference audio spectrums are obtained, and each reference audio spectrum is identified with an identification model generated by training, to determine a sleeping aid value of each reference audio spectrum. Then, reference audio spectrums are determined from the m reference audio spectrums as the plurality of sleeping aid audio spectrums, each of the reference audio spectrums corresponds to a sleeping aid value greater than a first threshold. And the plurality of sleeping aid audio spectrums are processed based on the sleeping aid values corresponding to the plurality of sleeping aid audio spectrums with the genetic algorithm, to obtain the sleeping aid audio spectrum chain. The sleeping aid audio is generated according to the sleeping aid audio spectrum chain. Therefore, the sleeping aid audio is generated with the genetic algorithm by taking the audio spectrum with relatively high sleeping aid value as the genetic material, so as to further improve the effect and reliability of the generated sleeping aid audio.

According to the invention, a plurality of sleeping aid audio spectrums are obtained, and the plurality of sleeping aid audio spectrums are processed with a genetic algorithm, to obtain a sleeping aid audio spectrum chain, and the sleeping aid audio is generated according to the sleeping aid audio spectrum chain. Sleep state parameters of a user are obtained, while the sleeping aid audio is played. The sleeping aid value of the sleeping aid audio is determined according to the sleep state parameters. In response to the sleeping aid value of the sleeping aid audio being less than a second threshold, a sleeping aid audio spectrum is removed from which the sleeping aid audio is generated from the plurality of sleeping aid audio spectrums, to obtain a plurality of sleeping aid audio spectrums updated. The sleeping aid audio corresponding to the user is generated by repeating above sleeping aid audio generation process based on the updated sleeping aid audio spectrum set. Thus, through the sleep state feedback of the user, the reliability and effectiveness of generating the sleeping aid audio are improved, personalized customization of the user is realized, and the sleeping aid audio with better sleep effect is generated scientifically and reliably.

It should be understood that the content described in this section is not intended to identify the key or important features of the embodiments of the disclosure, nor to limit the scope of the disclosure. Other features of the disclosure will be easily understood through the following instructions.

### BRIEF DESCRIPTING A THE DRAWINGS

Drawings are provided to better understand the present disclosure and are not intended to limit the present disclosure.
FIG. 1 is a schematic flowchart of a method not part of the claimed subject matter for generating sleeping aid audio provided by the present disclosure.
FIG. 2 is a schematic flowchart of another method not part of the claimed subject matter for generating sleeping aid audio provided by the present disclosure.
FIG. 3 is a schematic flowchart of another method for generating sleeping aid audio provided by the present disclosure.
FIG. 4 is a block diagram of an apparatus for generating a sleeping aid audio provided by the embodiments of the present disclosure.
FIG. 5 is a block diagram of an electronic device provided by the embodiments of the present disclosure.

### DETAILED DESCRIPTION

Embodiments of the disclosure are described as below with reference to the drawings, which include various details of embodiments of the disclosure to facilitate understanding and should be considered as merely example.

The method for generating sleeping aid audio proposed in the present disclosure can be implemented by an apparatus for generating sleeping aid audio provided in the present disclosure, or by an electronic device provided in the present disclosure. The electronic device includes, but not limited to, a terminal device, such as a desktop computer or a tablet computer, or a server. The following describes the method for generating sleeping aid audio provided in the present disclosure, which is implemented by the apparatus for generating sleeping aid audio provided in the present disclosure, not as a limitation of the disclosure, and the apparatus for generating sleeping aid audio provided in the present disclosure is hereinafter referred to as "the apparatus".

The following is a detailed description of the method, the apparatus, the computer device and storage medium for generating sleeping aid audio provided in the present disclosure.

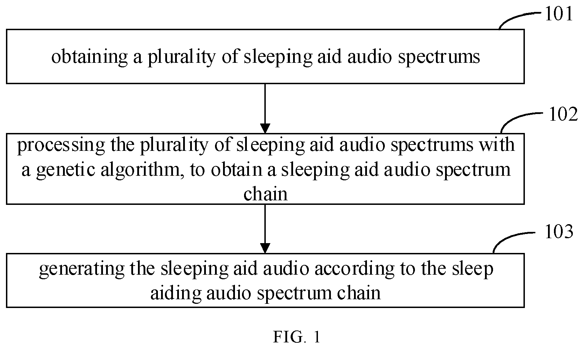

FIG. 1 is a schematic diagram of a method not part of the claimed subject matter for generating sleeping aid audio according to the embodiments of the present disclosure.

As illustrated in FIG. 1, the method for generating sleeping aid audio includes the following procedures S101 to S103.

At S101, a plurality of sleeping aid audio spectrums are obtained.

By combining melodies and music elements, sleeping aid audio may help people relieve stress and obtain physical and mental relaxation, so as to achieve a sleeping aid effect. Sleeping aid audio may be brain wave audio, binaural audio, audio played repeatedly at a fixed frequency, white noise or the like.

A sleeping aid audio spectrum refers to any type of audio spectrum with sleeping aid effect and can be processed by performing one or more operations of combination, variation and/or the like to generate audio.

At S102, the plurality of sleeping aid audio spectrums are processed with a genetic algorithm, to obtain a sleeping aid audio spectrum chain.

Specifically, after a sleeping aid audio spectrum set is obtained, each sleeping aid audio spectrum contained in the sleeping aid audio spectrum set can be used as a spectrum of the father generation, and then the genetic algorithm is performed on one or more spectrums of respective father generations. For example, the spectrums of multiple father generations are processed by crossover processing; or, one or more spectrums of any father generation are processed by mutation processing, and the result is then processed by cross-over processing with one or more spectrums of another father generation, so as to generate a new generation spectrum chain with the sleeping aid effect.

It should be noted that there are many ways of performing the crossover and mutation processing. The crossover processing includes single point crossover, multipoint crossover, uniform crossover, arithmetic crossover, etc. The mutation processing includes uniform mutation, boundary mutation, Gaussian approximation mutation, etc.

At S103, the sleeping aid audio is generated according to the sleeping aid audio spectrum chain.

Optionally, for the sleeping aid audio spectrum chain, inverse Fourier transform is performed to decode the sleeping aid audio spectrum chain to obtain the sleeping aid audio.

It should be noted that, in the method for generating the sleeping aid audio, a noise signal in the sleeping aid audio is optionally removed according to a frequency preset by a user.

In the embodiments of the present disclosure, a plurality of sleeping aid audio spectrums are obtained, and the plurality of sleeping aid audio spectrums are processed with the genetic algorithm, to obtain a sleeping aid audio spectrum chain. The sleeping aid audio is generated according to the sleeping aid audio spectrum chain. Therefore, the sleeping aid audio spectrums are used as genetic material to generate the sleeping aid audio with the genetic algorithm, which not only ensures an effect and reliability of sleeping aid audio, but also reduces a cost of sleeping aid audio.

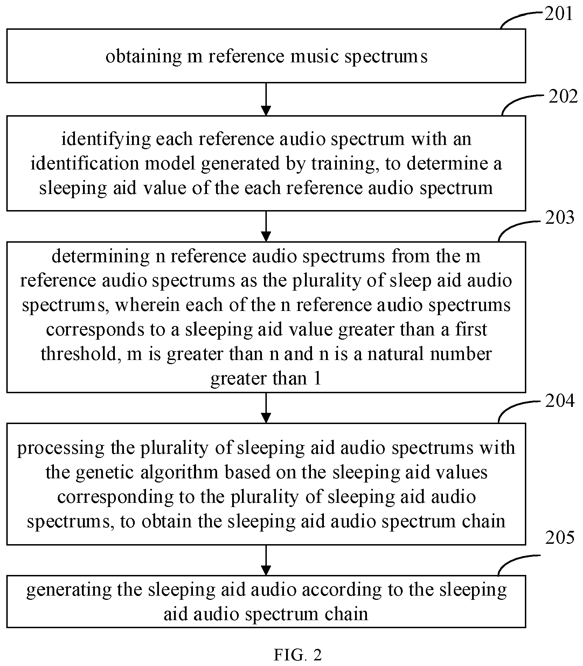

FIG. 2 is a schematic diagram of the method not part of the claimed subject matter for generating sleeping aid audio according to some other embodiments of the present disclosure.

As illustrated in FIG. 2, the method for generating the sleeping aid audio includes following procedures S201 to S205.

At S201, m reference audio spectrums are obtained, where m is a natural number greater than 1.

Optionally, the reference audio spectrums are obtained by processing original audio data of the reference audio. For example, for the original audio data of the reference audio, the original audio data is firstly segmented into audio segments with a fixed length, and the segments are then processed by Fourier transformation to obtain the reference audio spectrums corresponding to the audio segments.

It should be noted that, since sleeping aid audio may contain an audio segment with a fair sleeping aid effect, while non-sleeping-aid audio may contain an audio segment with relatively good sleeping aid effect. In order to better customize sleeping aid audio for the user to help the user achieve high-quality sleep in the present disclosure, a plurality types of reference audios are
selected from massive audio data in advance, and the reference audio can be any type.

The reference audio spectrums are optionally the audio spectrums containing sleeping aid audio and non-sleeping-aid audio, for example, the audio spectrum of the non-sleeping-aid audio such as heavy metals, rock and/or hip-hop, etc., and the audio spectrum of sleeping aid audio such as brain wave audio, binaural audio, audio with a fixed repetition frequency and/or white noise, etc.

At S202, each reference audio spectrum is identified with an identification model generated by training, to determine a sleeping aid value of the each reference audio spectrum.

The identification model is optionally obtained by training with labeled sleeping aid audio spectrums and non-sleeping-aid audio spectrums. The identification model generated by training can identify each reference audio spectrum. The identification model optionally has an architecture of a convolutional neural network or a recurrent neural network.

Specifically, after each reference audio spectrum is inputted into the identification model, the sleeping aid effect of the each reference audio spectrum is determined by the identification model. The output of the last layer of neurons in the identification model is optionally used as a sleeping aid value representing the sleeping aid effect of the sleeping aid audio spectrum.

At S203, n reference audio spectrums from the m reference audio spectrums are determined as the plurality of sleeping aid audio spectrums, where each of the n reference audio spectrums corresponds to a sleeping aid value greater than a first threshold, m is greater than n, and n is a natural number greater than 1.

The first threshold is optionally a preset sleeping aid threshold. If the sleeping aid value corresponding to a reference audio spectrum is greater than the first threshold, it indicates that the sleeping aid effect of this reference audio spectrum is relatively good. Therefore, this reference audio spectrum is determined as the sleeping aid audio spectrum.

For example, there are four reference audio spectrums, that is, n is 4, and the four reference audio spectrums are spectrum a, spectrum b, spectrum c and spectrum d, respectively. Spectrums a, b, c and d correspond to the sleeping aid values of 16, 36, 84 and 77, respectively. If the preset first threshold is 75, spectrum c and spectrum d in the reference audio spectrums are determined as the sleeping aid audio spectrums.

At S204, the plurality of sleeping aid audio spectrums are processed with a genetic algorithm based on the sleeping aid values corresponding to the plurality of sleeping aid audio spectrums, to obtain the sleeping aid audio spectrum chain.

In some embodiments, one or more target sleeping aid audio spectrums to be processed are selected from sleeping aid audio spectrums according to the sleeping aid values corresponding to the sleeping aid audio spectrums, and the one or more target sleeping aid audio spectrums are processed by a crossover operation and/or a mutation operation to generate a plurality of first-generation sleeping aid audio spectrums.

The one or more target sleeping aid audio spectrums are spectrums with relatively high sleeping aid values selected from the respective sleeping aid audio spectrums. By performing the crossover operation and/or the mutation operation on the target audio spectrums, a genetic and exploratory process is simulated to obtain the first-generation sleeping aid audio spectrums which are more suitable for sleeping aid. It should be noted that there are many ways of crossover operation and mutation operation. The crossover operation includes single point crossover, multipoint crossover, uniform crossover and/or arithmetic crossover, etc. The mutation operation includes uniform mutation, boundary mutation and/or Gaussian approximation mutation, etc.

Further, the target first-generation sleeping aid audio spectrums are selected from the plurality of first-generation sleeping aid audio spectrums according to the sleeping aid values corresponding to the first-generation sleeping aid audio spectrums. The crossover operation and/or the mutation operation are then performed repeatedly based on the target first-generation sleeping aid audio spectrums, until a number of operations performed reaches a preset value. A sleeping aid audio spectrum chain is determined based on the target sleeping aid audio spectrums and the generated respective generations of target sleeping aid audio spectrums.

It should be understood that, children audio spectrums with high fitness and good sleeping aid effect are selectively retained by performing the crossover operation and the mutation operation, namely, the first-generation sleeping aid audio spectrums. The target first-generation sleeping aid audio spectrums are the first-generation sleeping aid audio spectrums that better meets the requirements of sleeping aid. Based on the target first-generation sleeping aid audio spectrums, the crossover operation and/or the mutation operation are performed repeatedly to iterate and optimize constantly, so as to obtain the target sleeping aid audio spectrums, and thus the sleeping aid audio spectrum chain is obtained.

It should be noted that, in the method for generating the sleeping aid audio, when the genetic algorithm is used to process the sleeping aid audio spectrums, the sleeping aid values corresponding to the sleeping aid audio spectrums are used as fitness of the audio spectrums, and the one or more sleeping aid audio spectrums with sleeping aid values higher than a preset threshold are processed with the genetic algorithm. Thus, musical attributes of the sleeping aid audio generated tend to be more sleeping aid.

At S205, the sleeping aid audio is generated according to the sleeping aid audio spectrum chain.

It should be noted that, regarding the specific implementations of S205, the above embodiments can be referred to, and will not be illustrated in detail herein.

In the embodiments of the present disclosure, m reference audio spectrums are obtained, and each reference audio spectrum is identified with the identification model generated by training, to determine the sleeping aid value corresponding to the each reference audio spectrum. N reference audio spectrums are determined from the m reference audio spectrums as the plurality of sleeping aid audio spectrums, each of the n reference audio spectrums corresponds to a sleeping aid value greater than the first threshold. The plurality of sleeping aid audio spectrums are processed with the genetic algorithm based on the sleeping aid values corresponding to the plurality of sleeping aid audio spectrums, to obtain the sleeping aid audio spectrum chain. The sleeping aid audio is generated according to the sleeping aid audio spectrum chain. Therefore, the sleeping aid audio is generated with the genetic algorithm by taking the audio spectrum with relatively high sleeping aid value as the genetic material, so as to further improve the effect and reliability of the generated sleeping aid audio.

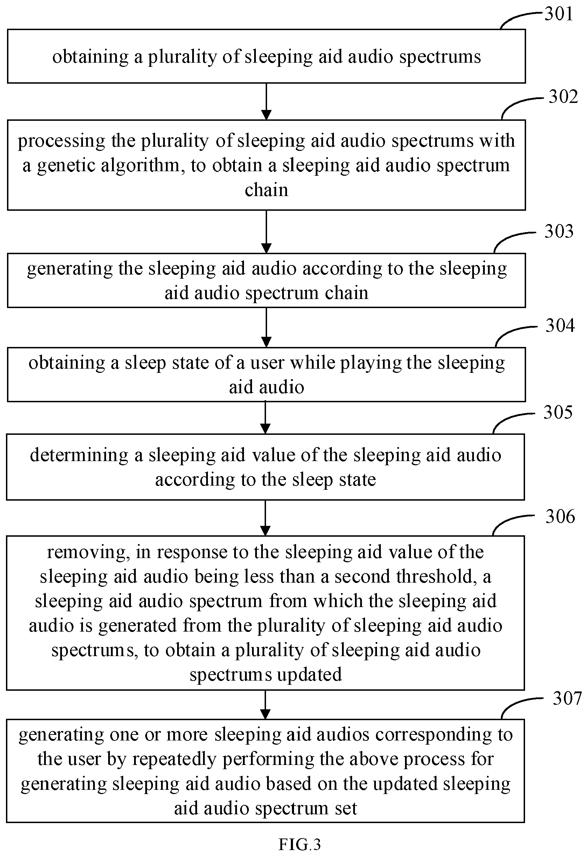

FIG. 3 is a schematic diagram of the method for generating a sleeping aid audio based on some other embodiments of the present disclosure.

As illustrated in FIG. 3, the method for generating the sleeping aid audio includes the following procedures S301 to S307.

At S301, a plurality of sleeping aid audio spectrums are obtained.

At S302, the plurality of sleeping aid audio spectrums are processed with a genetic algorithm, to obtain a sleeping aid audio spectrum chain.

At S303, the sleeping aid audio is generated according to the sleeping aid audio spectrum chain.

It should be noted that, the above embodiments can be referred to for the specific implementation process of S301, S302 and S303, which will not be illustrated in detail herein.

At S304, one or more sleep state parameters of the user are obtained while the sleeping aid audio is being played.

It should be noted that there are many ways to obtain the one or more sleep state parameters of the user. For example, a wearable device is used to collect a plurality of physiological parameters of the user, and the physiological parameters include at least one of the following parameters: a number of times of turn-overs, a heart rate, a blood pressure, a respiratory rate or a head motion frequency. The one or more sleep state parameters of the user are then determined according to the physiological parameters.

The plurality of physiological parameters of the user are collected by a wearable device, such as a watch or a bracelet. Specifically, the physiological parameters include the number of times of turn-overs, the heart rate, the blood pressure, the respiratory rate and/or the head motion frequency, etc.

The one or more sleep state parameters are used for representing a sleep state of the user, such as deep sleep, sound sleep, light sleep, falling asleep, etc.

Optionally, one or more thresholds of the physiological parameters are preset, such as a count threshold of turn-over, a heart rate threshold, a blood pressure threshold, a respiratory rate threshold and/or a head motion frequency threshold. The sleep state of the user is then inferred by comparing the physiological parameters with the respective physiological parameter thresholds. For example, if all of the number of times of turn-overs, the head motion frequency of the user and the heart rate of the user are lower than corresponding preset thresholds when the user is listening to sleeping aid audio A, it indicates that the user is currently in a sound sleep state.

A mapping relationship between physiological parameters and the sleep state parameters is optionally preset, and the current sleep state parameters of the user are then determined according to the physiological parameters collected during a specific sleeping aid audio is being played.

In some embodiments, the wearable device is configured to determine the sleep parameters of the user by monitoring spatial variations of a position and a posture of the user while the sleeping aid audio is being played.

It should be noted that, when a plurality of sleeping aid audios are played in sequence, one or more sleep state parameters of the user corresponding to each sleeping aid audio are optionally obtained.

At S305, the sleeping aid values of the one or more sleeping aid audio spectrums for generating the one or more sleeping aid audios are determined according to the sleep state parameters.

An influence degree of each sleeping aid audio on the sleep state of the user, namely, the sleeping aid effect, is determined through the sleep state parameters.

Optionally, the sleep state parameters are inputted into a pre-trained sleeping aid identification network, so that one or more sleeping aid values corresponding to the respective sleep state parameters corresponding to the one or more sleeping aid audios are determined. The sleeping aid identification network can be realized by various deep neural networks (DNN).

At S306, in response to the sleeping aid value of a sleeping aid audio being less than a second threshold, a sleeping aid audio spectrum for generating the sleeping aid audio is removed from the plurality of sleeping aid audio spectrums, to obtain a plurality of sleeping aid audio spectrums updated.

It should be understood that, an effect of the sleeping aid audio is obtained by comparing the sleeping aid value of the sleeping aid audio with the second threshold. For example, if the sleeping aid value of the sleeping aid audio is higher than the second threshold, it indicates that the current sleeping aid audio is the sleeping aid audio with high fitness and relatively good sleeping aid ability. The sleeping aid audio spectrum set is updated by removing the sleeping aid audio spectrum generating the sleeping aid audio with a sleeping aid value lower than the second threshold, so that the updated sleeping aid audio spectrum set is more suitable for the user.

At S307, one or more sleeping aid audios corresponding to the user are generated by repeatedly performing the above process for generating sleeping aid audio based on the updated sleeping aid audio spectrum set.

Specifically, by a continuous repetition of the process for generating the sleeping aid audio, the sleeping aid audio spectrum set is continuously adapted to sleep characteristics of the user, so as to rapidly adapt and meet the requirements of the user.

In some embodiments of the present disclosure, a plurality of sleeping aid audio spectrums are obtained, and the plurality of sleeping aid audio spectrums are processed with a genetic algorithm, to obtain a sleeping aid audio spectrum chain. Sleep state parameters of a user are obtained while the sleeping aid audio is being played. The sleeping aid value of the sleeping aid audio is determined according to the sleep state parameters. In response to the sleeping aid value of one sleeping aid audio being less than a second threshold, a sleeping aid audio spectrum from which the one sleeping aid audio is generated is removed from the plurality of sleeping aid audio spectrums, so as to obtain a plurality of sleeping aid audio spectrums updated. One or more sleeping aid audios corresponding to the user are generated by repeatedly performing the above process for generating the sleeping aid audio based on the updated sleeping aid audio spectrum set. Therefore, through the sleep state feedback of the user, the reliability and effectiveness of generation of the sleeping aid audio are improved, personalized customization for the user is realized, and the sleeping aid audio with better sleep effect is generated scientifically and reliably.

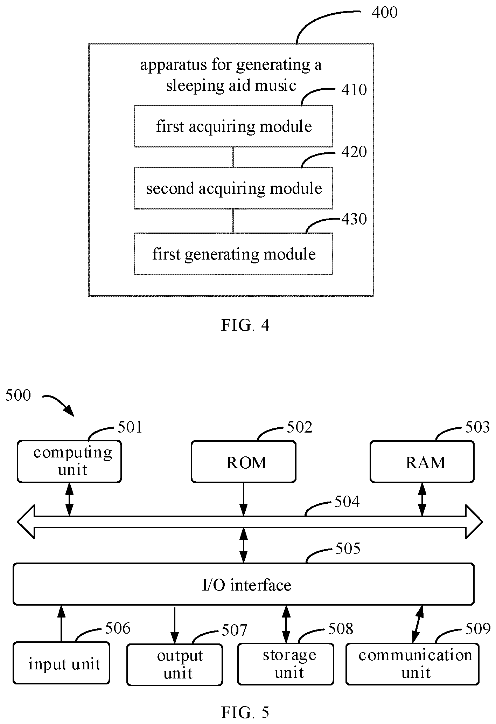

In order to realize the above implementations, some embodiment of the present disclosure also provide an apparatus for generating sleeping aid audio. Fig. 4 illustrates a structural diagram of an apparatus for generating sleeping aid audio provided by some embodiments of the present disclosure.

As illustrated in Fig. 4, the apparatus for generating the sleeping aid audio includes: a first acquiring module 410, a second acquiring module 420 and a first generating module 430.

The first acquiring module 410 is configured to obtain a plurality of sleeping aid audio spectrums.

The second acquiring module 420 is configured to process the plurality of sleeping aid audio spectrums with a genetic algorithm, to obtain a sleeping aid audio spectrum chain.

The first generating module 430 is configured to generate the sleeping aid audio according to the sleeping aid audio spectrum chain.

In some embodiments, the first acquiring module 410 is specifically configured to: obtain m reference audio spectrums; identify each reference audio spectrum with an identification model generated by training, to determine a sleeping aid value of the each reference audio spectrum; and determine n reference audio spectrums from the m reference audio spectrums as the sleeping aid audio spectrums. Each of the n reference audio spectrums corresponds to a sleeping aid value greater than a first threshold, m is greater than n and n is a natural number greater than 1.

In some embodiments, the second acquiring module 420 includes a processing unit, configured to process the plurality of sleeping aid audio spectrums with the genetic algorithm based on the sleeping aid values corresponding to the plurality of sleeping aid audio spectrums, to obtain the sleeping aid audio spectrum chain.

In some embodiments, the processing unit is specifically configured to select one or more target sleeping aid audio spectrums to be processed from the plurality of sleeping aid audio spectrums based on the sleeping aid values corresponding to the plurality of sleeping aid audio spectrums; process the one or more target sleeping aid audio spectrums by performing at least one of a crossover operation and a mutation operation, to generate a plurality of first-generation sleeping aid audio spectrums; select one or more target first-generation sleeping aid audio spectrums from the plurality of first-generation sleeping aid audio spectrums based on the sleeping aid values corresponding to the plurality of first-generation sleeping aid audio spectrums; and preform, repeatedly until a number of operations performed reaches a preset value, at least one of the crossover operation and the mutation operation based on the one or more target first-generation sleeping aid audio spectrums, and determine the sleeping aid audio spectrum chain according to the one or more target sleeping aid audio spectrums and generated respective generations of target sleeping aid audio spectrums.

In some embodiments, the first generating module 430 includes a first acquiring unit, a first generating unit and a second generating unit.

The first acquiring unit is configured to obtain a sleep state of a user while the sleeping aid audio is being played.

The first generating unit is configured to determine the sleeping aid value of the sleeping aid audio according to the sleep state.

The second generating unit is configured to remove a sleeping aid audio spectrum from which the sleeping aid audio is generated from the plurality of sleeping aid audio spectrums, to obtain a plurality of sleeping aid audio spectrums updated, in response to the sleeping aid value of the sleeping aid audio being less than a second threshold.

In some embodiments, the first acquiring unit is configured to obtain a plurality of physiological parameters of a user collected by the wearable device, the plurality of physiological parameters include at least one of: a number of times of turn-overs, a heart rate, a blood pressure, a respiratory rate or a head motion frequency; and determine the sleep state of the user according to the plurality of physiological parameters.

In an embodiment of the present disclosure, the apparatus may obtain a plurality of sleeping aid audio spectrums, and processes the plurality of sleeping aid audio spectrums with a genetic algorithm, to obtain a sleeping aid audio spectrum chain. The sleeping aid audio is generated based on the sleeping aid audio spectrum chain. Therefore, the sleeping aid audio spectrums may be used as genetic material to generate sleeping aid audio by using the genetic algorithm, which not only ensures an effect and reliability of sleeping aid audio, but also reduces a cost of sleeping aid audio.

According to an embodiment of the present disclosure, a wearable device, a readable storage medium and a computer program product are provided.

FIG. 5 illustrates a schematic diagram of an electronic device 500 which may be used to implement embodiments of the present disclosure. Electronic device aims to represent various forms of digital computers, such as laptop computers, desktop computers, worktables, personal digital assistants, servers, blade servers, large computers, and other suitable computers. An electronic device may also represent various forms of mobile devices, such as personal digital processors, cellular phones, smart phones, wearable devices and other similar computing devices. The components shown herein, their connections and relations, and their functions are merely examples, and are not intended to limit the implementation of the disclosure described and/or required herein.

As illustrated in FIG. 5, the device 500 includes a computing unit 501, which may execute various appropriate actions and processes based on the computer program stored in the read-only memory (ROM) 502 or a computer program loaded a random access memory (RAM) 503 from a storage unit 508. In the RAM 503, various programs and data required for operation of the device 500 may also be stored. The computing unit 501, the ROM 502 and the RAM 503 are connected to each other through a bus 504. An input/output (I/O) interface 505 is also connected to the bus 504.

Several components in the device 500 are connected to the I/O interface 505, and include: an input unit 506, for example, keyboard, mouse, etc. an output unit 507, for example, various types of displays, speakers; a storage unit 508, for example, a magnetic disk, an optical disk, etc.; and a communication unit 509, for example, a network card, a modem, a wireless communication transceiver, etc. The communication unit 509 allows a device 500 to exchange information/data through a computer network such as internet and/or various types of telecommunication networks and other devices.

The computing unit 501 may be various general-purpose and/or special-purpose processing components with processing and computing capacities. Some examples of the computing unit 501 include but are not limited to a central processing unit (CPU), a graphics processing units (GPU), various dedicated artificial intelligence (AI) computing chips, various computing units running a machine learning model algorithm, a digital signal processors (DSP), and any appropriate processor, controller, microcontroller, etc. The computing unit 501 executes various methods and processes as described above, for example, in some embodiments, the method for generating the sleeping aid audio may be achieved as a computer software program, which is physically contained in a machine readable medium, such as a storage unit 508. In some embodiments, a part or all of the computer program may be loaded and/or installed on the device 500 via a ROM 502 and/or a communication unit 509. When the computer program is loaded on a RAM 503 and executed by a computing unit 501, one or more blocks in the method for generating sleeping aid audio described above may be performed. In some embodiments, the computing unit 501 may be configured to perform a method for generating a sleeping aid audio in any other appropriate ways (for example, virtue of a firmware).

Various implementation modes of the systems and technologies described above may be achieved in a digital electronic circuit system, a field programmable gate array (FPGA), an application-specific integrated circuit (ASIC), an application specific standard product (ASSP), a system-on-chip (SOC) system, a complex programmable logic device, a computer hardware, a firmware, a software, and/or combinations thereof. The various implementation modes may include: implementation in one or more computer programs, and the one or more computer programs may be executed and/or interpreted on a programmable system including at least one programmable processor, and the programmable processor may be a dedicated or a general-purpose programmable processor that may receive data and instructions from a storage system, at least one input apparatus, and at least one output apparatus, and transmit the data and instructions to the storage system, the at least one input apparatus, and the at least one output apparatus.

A computer code configured to execute a method in the present disclosure may be written with one or any combination of a plurality of programming languages. The programming languages may be provided to a processor or a controller of a general purpose computer, a dedicated computer, or other apparatuses for programmable data processing so that the function/operation specified in the flowchart and/or block diagram may be performed when the program code is executed by the processor or controller. A computer code may be performed completely or partly on the machine, performed partly on the machine as an independent software package and performed partly or completely on the remote machine or server.

In the context of the disclosure, a machine-readable medium may be a tangible medium that may contain or store a program intended for use in or in conjunction with an instruction execution system, apparatus, or device. A machine readable medium may be a machine readable signal medium or a machine readable storage medium. A machine readable storage medium may include but not limited to an electronic, magnetic, optical, electromagnetic, infrared, or semiconductor system, apparatus or device, or any appropriate combination thereof. A more specific example of a machine readable storage medium includes an electronic connector with one or more cables, a portable computer disk, a hardware, a random access memory (RAM), a read-only memory (ROM), an erasable programmable read-only memory (an EPROM or a flash memory), an optical fiber device, and a portable optical disk read-only memory (CDROM), an optical storage device, a magnetic storage device, or any appropriate combination of the above.

In order to provide interaction with the user, the systems and technologies described here may be implemented on a computer, and the computer has: a display apparatus for displaying information to the user (for example, a CRT (cathode ray tube) or an LCD (liquid crystal display) monitor); and a keyboard and a pointing apparatus (for example, a mouse or a trackball) through which the user may provide input to the computer. Other types of apparatuses may further be configured to provide interaction with the user; for example, the feedback provided to the user may be any form of sensory feedback (for example, visual feedback, auditory feedback, or tactile feedback); and input from the user may be received in any form (including an acoustic input, a speech input, or a tactile input).

The systems and technologies described herein may be implemented in a computing system including back-end components (for example, as a data server), or a computing system including middleware components (for example, an application server), or a computing system including front-end components (for example, a user computer with a graphal user interface or a web browser through which the user may interact with the implementation mode of the system and technology described herein), or a computing system including any combination of such back-end components, middleware components or front-end components. The system components may be connected to each other through any form or medium of digital data communication (for example, a communication network). Examples of communication networks include: a local area network (LAN), a wide area network (WAN), an internet and a blockchain network.

The computer system may include a client and a server. The client and server are generally far away from each other and generally interact with each other through a communication network. The relationship between the client and the server is generated by computer programs running on the corresponding computer and having a client-server relationship with each other. A server may be a cloud server, also known as a cloud computing server or a cloud host, is a host product in a cloud computing service system, to solve the shortcomings of large management difficulty and weak business expansibility existed in the conventional physical host and Virtual Private Server (VPS) service. A server further may be a server with a distributed system, or a server in combination with a block chain.

In an embodiment of the present disclosure, a plurality of sleeping aid audio spectrums are obtained, and the plurality of sleeping aid audio spectrums are processed with a genetic algorithm, to obtain a sleeping aid audio spectrum chain. The sleeping aid audio is generated based on the sleeping aid audio spectrum chain. Therefore, the sleeping aid audio spectrums may be used as genetic material to generate sleeping aid audio by using the genetic algorithm, which not only ensures an effect and reliability of sleeping aid audio, but also reduces a cost of sleeping aid audio.

## Claims

1. A method for generating a sleeping aid audio, comprising:
obtaining (101) a plurality of sleeping aid audio spectrums;
processing (102) the plurality of sleeping aid audio spectrums with a genetic algorithm, to obtain a sleeping aid audio spectrum chain;
generating (103) the sleeping aid audio according to the sleeping aid audio spectrum chain;
wherein the method further comprises:
obtaining one or more sleep state parameters of a user while playing the sleeping aid audio;
determining a sleeping aid value of the sleeping aid audio according to the sleep state parameters and a pre-trained sleeping aid identification network; and
removing (306), in response to the sleeping aid value of the sleeping aid audio being less than a threshold, a sleeping aid audio spectrum from which the sleeping aid audio is generated from the plurality of sleeping aid audio spectrums, to obtain a plurality of sleeping aid audio spectrums updated.

2. The method of claim 1, wherein obtaining (101) the plurality of sleeping aid audio spectrums comprises:
obtaining (201) m reference audio spectrums;
identifying (202) each reference audio spectrum with an identification model generated by training, to determine a sleeping aid value of the each reference audio spectrum; and
determining (203) n reference audio spectrums from the m reference audio spectrums as the plurality of sleeping aid audio spectrums, wherein each of the n reference audio spectrums corresponds to a sleeping aid value greater than a further threshold, m is greater than n and n is a natural number greater than 1.

3. The method of claim 2, wherein processing (102) the plurality of sleeping aid audio spectrums with the genetic algorithm, to obtain the sleeping aid audio spectrum chain comprises:
processing (204) the plurality of sleeping aid audio spectrums with the genetic algorithm based on the sleeping aid values corresponding to the plurality of sleeping aid audio spectrums, to obtain the sleeping aid audio spectrum chain.

4. The method of claim 3, wherein processing (102) the plurality of sleeping aid audio spectrums with the genetic algorithm based on the sleeping aid values corresponding to the plurality of sleeping aid audio spectrums, to obtain the sleeping aid audio spectrum chain comprises:
selecting one or more target sleeping aid audio spectrums to be processed from the plurality of sleeping aid audio spectrums based on the sleeping aid values corresponding to the plurality of sleeping aid audio spectrums;
processing the one or more target sleeping aid audio spectrums by performing at least one of a crossover operation and a mutation operation, to generate a plurality of first-generation sleeping aid audio spectrums;
selecting one or more target first-generation sleeping aid audio spectrums from the plurality of first-generation sleeping aid audio spectrums based on sleeping aid values corresponding to the plurality of first-generation sleeping aid audio spectrums; and
performing, repeatedly until a number of operations performed reaches a preset value, at least one of the crossover operation and the mutation operation based on the one or more target first-generation sleeping aid audio spectrums, and
determining the sleeping aid audio spectrum chain according to the one or more target sleeping aid audio spectrums and generated respective generations of target sleeping aid audio spectrums.

5. The method of claim 1, wherein obtaining the sleep state of the user comprises:
obtaining a plurality of physiological parameters of the user collected by a wearable device, wherein the plurality of physiological parameters comprise at least one of: a number of times of turn-overs, a heart rate, a blood pressure, a respiratory rate and a head motion frequency; and
determining the one or more sleep state parameter of the user according to the plurality of physiological parameters.

6. An apparatus (400) for generating a sleeping aid audio, comprising:
a first acquiring module, configured to obtain a plurality of sleeping aid audio spectrums;
a second acquiring module (410), configured to process the plurality of sleeping aid audio spectrums with a genetic algorithm, to obtain a sleeping aid audio spectrum chain; and
a first generating module (420), configured to generate the sleeping aid audio according to the sleeping aid audio spectrum chain;
wherein the first generating module (430) comprises:
a first acquiring unit, configured to obtain one or more sleep state parameters of a user while playing the sleeping aid audio;
a first generating unit, configured to determine a sleeping aid value of the sleeping aid audio according to the sleep state parameters and a pre-trained sleeping aid identification network; and
a second generating unit, configured to remove, in response to the sleeping aid value of the sleeping aid audio being less than a second threshold, a sleeping aid audio spectrum from which the sleeping aid audio is generated from the plurality of sleeping aid audio spectrums, to obtain a plurality of sleeping aid audio spectrums updated.

7. The apparatus in claim 6, wherein the first acquiring module (410) is configured to:
obtain m reference audio spectrums;
identify each reference audio spectrum with an identification model generated by training, to determine a sleeping aid value of each reference audio spectrum; and
determine n reference audio spectrums from the m reference audio spectrums as the plurality of sleeping aid audio spectrums, wherein each of the n reference audio spectrums corresponds to a sleeping aid value greater than a first threshold, m is greater than n and n is a natural number greater than 1.

8. The apparatus (400) in claim 7, wherein the second acquiring module (420) comprises:
a processing unit, configured to process the plurality of sleeping aid audio spectrums with the genetic algorithm based on the sleeping aid values corresponding to the plurality of sleeping aid audio spectrums, to obtain the sleeping aid audio spectrum chain.

9. The apparatus (400) in claim 8, wherein the processing unit is configured to:
select one or more target sleeping aid audio spectrums to be processed from the plurality of sleeping aid audio spectrums based on the sleeping aid values corresponding to the plurality of sleeping aid audio spectrums;
process the one or more target sleeping aid audio spectrums by performing at least one of a crossover operation and a mutation operation, to generate a plurality of first-generation sleeping aid audio spectrums;
select one or more target first-generation sleeping aid audio spectrums from the plurality of first-generation sleeping aid audio spectrums based on sleeping aid values corresponding to the plurality of first-generation sleeping aid audio spectrums; and
perform, repeatedly until a number of operations performed reaches a preset value, at least one of the crossover operation and the mutation operation based on the one or more target first-generation sleeping aid audio spectrums, and
determine the sleeping aid audio spectrum chain according to the one or more target sleeping aid audio spectrums and generated respective generations of target sleeping aid audio spectrums.

10. The apparatus (400) in claim 6, wherein the first acquiring unit is configured to:
obtain a plurality of physiological parameters of the user collected by a wearable device, wherein the plurality of physiological parameters comprise at least one of: a number of times of turn-overs, a heart rate, a blood pressure, a respiratory rate or a head motion frequency; and
determine the one or more sleep state parameters of the user according to the plurality of physiological parameters.

11. An electronic device, comprising:
at least one processor; and
a memory communicatively connected to the at least one processor; wherein
the memory stores instructions that are executable by the at least one processor, and execution of the instructions by the at least one processor causes the at least one processor to perform the method of any one of claims 1 to 5.

12. A non-transitory computer readable storage medium, having computer instructions stored thereon, wherein the computer instructions are configured to cause a computer to perform the method of any one of claims 1 to 5.

13. A computer program product comprising a computer program, wherein during execution of the computer program by a processor, the method of any one of claims 1 to 5 is performed.

## Patentansprüche

1. Verfahren zum Erzeugen eines Schlafhilfeaudios, umfassend:
Erhalten (101) einer Vielzahl von Schlafhilfeaudiospektren;
Verarbeiten (102) der Vielzahl von Schlafhilfeaudiospektren mit einem genetischen Algorithmus, um eine Schlafhilfeaudiospektrumkette zu erhalten;
Erzeugen (103) des Schlafhilfeaudios gemäß der Schlafhilfeaudiospektrumkette;
wobei das Verfahren ferner umfasst:
Erhalten eines oder mehrerer Schlafzustandsparameter eines Benutzers während des Abspielens des Schlafhilfeaudios;
Bestimmen eines Schlafhilfewerts des Schlafhilfeaudios gemäß den Schlafzustandsparametern und einem vortrainierten Schlafhilfeidentifikationsnetzwerk; und
Entfernen (306), als Reaktion darauf, dass der Schlafhilfewert des Schlafhilfeaudios kleiner als ein Schwellenwert ist, eines Schlafhilfeaudiospektrums, aus dem das Schlafhilfeaudio erzeugt wird, aus der Vielzahl von Schlafhilfeaudiospektren, um eine Vielzahl von aktualisierten Schlafhilfeaudiospektren zu erhalten.

2. Verfahren nach Anspruch 1, wobei das Erhalten (101) der Vielzahl von Schlafhilfeaudiospektren umfasst:
Erhalten (201) von m Referenzaudiospektren;
Identifizieren (202) jedes Referenzaudiospektrums mit einem durch Training erzeugten Identifikationsmodell, um einen Schlafhilfewert jedes Referenzaudiospektrums zu bestimmen; und
Bestimmen (203) von n Referenzaudiospektren aus den m Referenzaudiospektren als die Vielzahl von Schlafhilfeaudiospektren, wobei jedes der n Referenzaudiospektren einem Schlafhilfewert entspricht, der größer als ein weiterer Schwellenwert ist, m größer als n ist und n eine natürliche Zahl größer als 1 ist.

3. Verfahren nach Anspruch 2, wobei das Verarbeiten (102) der Vielzahl von Schlafhilfeaudiospektren mit dem genetischen Algorithmus, um die Schlafhilfeaudiospektrumkette zu erhalten, umfasst:
Verarbeiten (204) der Vielzahl von Schlafhilfeaudiospektren mit dem genetischen Algorithmus basierend auf den Schlafhilfewerten, die der Vielzahl von Schlafhilfeaudiospektren entsprechen, um die Schlafhilfeaudiospektrumkette zu erhalten.

4. Verfahren nach Anspruch 3, wobei das Verarbeiten (102) der Vielzahl von Schlafhilfeaudiospektren mit dem genetischen Algorithmus basierend auf den Schlafhilfewerten, die der Vielzahl von Schlafhilfeaudiospektren entsprechen, um die Schlafhilfeaudiospektrumkette zu erhalten, umfasst:
Auswählen eines oder mehrerer zu verarbeitender Zielschlafhilfeaudiospektren aus der Vielzahl von Schlafhilfeaudiospektren basierend auf den Schlafhilfewerten, die der Vielzahl von Schlafhilfeaudiospektren entsprechen;
Verarbeiten des einen oder der mehreren Zielschlafhilfeaudiospektren durch Durchführen mindestens einer von einer Crossover-Operation und einer Mutationsoperation, um eine Vielzahl von Schlafhilfeaudiospektren der ersten Generation zu erzeugen;
Auswählen eines oder mehrerer Zielschlafhilfeaudiospektren der ersten Generation aus der Vielzahl von Schlafhilfeaudiospektren der ersten Generation basierend auf Schlafhilfewerten, die der Vielzahl von Schlafhilfeaudiospektren der ersten Generation entsprechen; und
wiederholtes Durchführen, bis eine Anzahl von durchgeführten Operationen einen voreingestellten Wert erreicht, mindestens einer von der Crossover-Operation und der Mutationsoperation basierend auf dem einen oder den mehreren Zielschlafhilfeaudiospektren der ersten Generation, und
Bestimmen der Schlafhilfeaudiospektrumkette gemäß dem einen oder den mehreren Zielschlafhilfeaudiospektren und erzeugten jeweiligen Generationen von Zielschlafhilfeaudiospektren.

5. Verfahren nach Anspruch 1, wobei das Erhalten des Schlafzustands des Benutzers umfasst:
Erhalten einer Vielzahl von physiologischen Parametern des Benutzers, die von einer tragbaren Vorrichtung gesammelt werden, wobei die Vielzahl von physiologischen Parametern mindestens eines umfasst von: einer Anzahl von Lagewechseln, einer Herzfrequenz, einem Blutdruck, einer Atemfrequenz und einer Kopfbewegungsfrequenz; und
Bestimmen des einen oder der mehreren Schlafzustandsparameter des Benutzers gemäß der Vielzahl von physiologischen Parametern.

6. Einrichtung (400) zum Erzeugen eines Schlafhilfeaudios, umfassend:
ein erstes Erfassungsmodul, das konfiguriert ist, um eine Vielzahl von Schlafhilfeaudiospektren zu erhalten;
ein zweites Erfassungsmodul (410), das konfiguriert ist, um die Vielzahl von Schlafhilfeaudiospektren mit einem genetischen Algorithmus zu verarbeiten, um eine Schlafhilfeaudiospektrumkette zu erhalten; und
ein erstes Erzeugungsmodul (420), das konfiguriert ist, um das Schlafhilfeaudio gemäß der Schlafhilfeaudiospektrumkette zu erzeugen;
wobei das erste Erzeugungsmodul (430) umfasst:
eine erste Erfassungseinheit, die konfiguriert ist, um einen oder mehrere Schlafzustandsparameter eines Benutzers während des Abspielens des Schlafhilfeaudios zu erhalten;
eine erste Erzeugungseinheit, die konfiguriert ist, um einen Schlafhilfewert des Schlafhilfeaudios gemäß den Schlafzustandsparametern und einem vortrainierten Schlafhilfeidentifikationsnetzwerk zu bestimmen; und
eine zweite Erzeugungseinheit, die konfiguriert ist, um als Reaktion darauf, dass der Schlafhilfewert des Schlafhilfeaudios kleiner als ein zweiter Schwellenwert ist, ein Schlafhilfeaudiospektrum, aus dem das Schlafhilfeaudio erzeugt wird, aus der Vielzahl von Schlafhilfeaudiospektren zu entfernen, um eine Vielzahl von aktualisierten Schlafhilfeaudiospektren zu erhalten.

7. Einrichtung nach Anspruch 6, wobei das erste Erfassungsmodul (410) konfiguriert ist zum:
Erhalten von m Referenzaudiospektren;
Identifizieren jedes Referenzaudiospektrums mit einem durch Training erzeugten Identifikationsmodell, um einen Schlafhilfewert jedes Referenzaudiospektrums zu bestimmen; und
Bestimmen von n Referenzaudiospektren aus den m Referenzaudiospektren als die Vielzahl von Schlafhilfeaudiospektren, wobei jedes der n Referenzaudiospektren einem Schlafhilfewert entspricht, der größer als ein erster Schwellenwert ist, m größer als n ist und n eine natürliche Zahl größer als 1 ist.

8. Einrichtung (400) nach Anspruch 7, wobei das zweite Erfassungsmodul (420) umfasst:
eine Verarbeitungseinheit, die konfiguriert ist, um die Vielzahl von Schlafhilfeaudiospektren mit dem genetischen Algorithmus basierend auf den Schlafhilfewerten, die der Vielzahl von Schlafhilfeaudiospektren entsprechen, zu verarbeiten, um die Schlafhilfeaudiospektrumkette zu erhalten.

9. Einrichtung (400) nach Anspruch 8, wobei die Verarbeitungseinheit konfiguriert ist zum:
Auswählen eines oder mehrerer zu verarbeitender Zielschlafhilfeaudiospektren aus der Vielzahl von Schlafhilfeaudiospektren basierend auf den Schlafhilfewerten, die der Vielzahl von Schlafhilfeaudiospektren entsprechen;
Verarbeiten des einen oder der mehreren Zielschlafhilfeaudiospektren durch Durchführen mindestens einer von einer Crossover-Operation und einer Mutationsoperation, um eine Vielzahl von Schlafhilfeaudiospektren der ersten Generation zu erzeugen;
Auswählen eines oder mehrerer Zielschlafhilfeaudiospektren der ersten Generation aus der Vielzahl von Schlafhilfeaudiospektren der ersten Generation basierend auf Schlafhilfewerten, die der Vielzahl von Schlafhilfeaudiospektren der ersten Generation entsprechen; und
wiederholtes Durchführen, bis eine Anzahl von durchgeführten Operationen einen voreingestellten Wert erreicht, mindestens einer von der Crossover-Operation und der Mutationsoperation basierend auf dem einen oder den mehreren Zielschlafhilfeaudiospektren der ersten Generation, und
Bestimmen der Schlafhilfeaudiospektrumkette gemäß dem einen oder den mehreren Zielschlafhilfeaudiospektren und erzeugten jeweiligen Generationen von Zielschlafhilfeaudiospektren.

10. Einrichtung (400) nach Anspruch 6, wobei die erste Erfassungseinheit konfiguriert ist zum:
Erhalten einer Vielzahl von physiologischen Parametern des Benutzers, die von einer tragbaren Vorrichtung gesammelt werden, wobei die Vielzahl von physiologischen Parametern mindestens eines umfasst von: einer Anzahl von Malen von Umdrehungen, einer Herzfrequenz, einem Blutdruck, einer Atemfrequenz oder einer Kopfbewegungsfrequenz; und
Bestimmen des einen oder der mehreren Schlafzustandsparameter des Benutzers gemäß der Vielzahl von physiologischen Parametern.

11. Elektronische Vorrichtung, umfassend:
mindestens einen Prozessor; und
einen Speicher, der kommunikativ mit dem mindestens einen Prozessor verbunden ist; wobei
der Speicher Anweisungen speichert, die durch den mindestens einen Prozessor ausführbar sind, und die Ausführung der Anweisungen durch den mindestens einen Prozessor den mindestens einen Prozessor veranlasst, das Verfahren nach einem der Ansprüche 1 bis 5 durchzuführen.

12. Nichtflüchtiges computerlesbares Speichermedium, auf dem Computeranweisungen gespeichert sind, wobei die Computeranweisungen konfiguriert sind, um einen Computer zu veranlassen, das Verfahren nach einem der Ansprüche 1 bis 5 durchzuführen.

13. Computerprogrammprodukt, umfassend ein Computerprogramm, wobei während der Ausführung des Computerprogramms durch einen Prozessor das Verfahren nach einem der Ansprüche 1 bis 5 durchgeführt wird.

## Revendications

1. Procédé de génération d'un audio d'aide au sommeil, comprenant:
l'obtention (101) d'une pluralité de spectres audio d'aide au sommeil;
le traitement (102) de la pluralité de spectres audio d'aide au sommeil avec un algorithme génétique, pour obtenir une chaîne de spectres audio d'aide au sommeil ;
la génération (103) de l'audio d'aide au sommeil selon la chaîne de spectres audio d'aide au sommeil;
dans lequel le procédé comprend en outre:
l'obtention d'un ou plusieurs paramètres d'état de sommeil d'un utilisateur pendant la lecture de l'audio d'aide au sommeil;
la détermination d'une valeur d'aide au sommeil de l'audio d'aide au sommeil selon les paramètres d'état de sommeil et un réseau d'identification d'aide au sommeil pré - entraîné; et
la suppression (306), en réponse au fait que la valeur d'aide au sommeil de l'audio d'aide au sommeil est inférieure à un seuil, d'un spectre audio d'aide au sommeil à partir duquel l'audio d'aide au sommeil est généré à partir de la pluralité de spectres audio d'aide au sommeil, pour obtenir une pluralité de spectres audio d'aide au sommeil mis à jour.

2. Procédé selon la revendication 1, dans lequel l'obtention (101) de la pluralité de spectres audio d'aide au sommeil comprend:
l'obtention (201) de m spectres audio de référence;
l'identification (202) de chaque spectre audio de référence avec un modèle d'identification généré par entraînement, pour déterminer une valeur d'aide au sommeil de chaque spectre audio de référence; et
la détermination (203) de n spectres audio de référence à partir des m spectres audio de référence en tant que pluralité de spectres audio d'aide au sommeil, dans lequel chacun des n spectres audio de référence correspond à une valeur d'aide au sommeil supérieure à un autre seuil, m est supérieur à n et n est un nombre naturel supérieur à 1.

3. Procédé selon la revendication 2, dans lequel le traitement (102) de la pluralité de spectres audio d'aide au sommeil avec l'algorithme génétique, pour obtenir la chaîne de spectres audio d'aide au sommeil comprend:
le traitement (204) de la pluralité de spectres audio d'aide au sommeil avec l'algorithme génétique sur la base des valeurs d'aide au sommeil correspondant à la pluralité de spectres audio d'aide au sommeil, pour obtenir la chaîne de spectres audio d'aide au sommeil.

4. Procédé selon la revendication 3, dans lequel le traitement (102) de la pluralité de spectres audio d'aide au sommeil avec l'algorithme génétique sur la base des valeurs d'aide au sommeil correspondant à la pluralité de spectres audio d'aide au sommeil, pour obtenir la chaîne de spectres audio d'aide au sommeil comprend:
la sélection d'un ou plusieurs spectres audio d'aide au sommeil cibles à traiter parmi la pluralité de spectres audio d'aide au sommeil sur la base des valeurs d'aide au sommeil correspondant à la pluralité de spectres audio d'aide au sommeil;
le traitement des un ou plusieurs spectres audio d'aide au sommeil cibles en effectuant au moins l'une d'une opération de croisement et d'une opération de mutation, pour générer une pluralité de spectres audio d'aide au sommeil de première génération ;
la sélection d'un ou plusieurs spectres audio d'aide au sommeil cibles de première génération parmi la pluralité de spectres audio d'aide au sommeil de première génération sur la base de valeurs d'aide au sommeil correspondant à la pluralité de spectres audio d'aide au sommeil de première génération; et
l'exécution, de manière répétée jusqu'à ce qu'un nombre d'opérations effectuées atteigne une valeur prédéfinie, d'au moins l'une de l'opération de croisement et de l'opération de mutation sur la base des un ou plusieurs spectres audio d'aide au sommeil cibles de première génération, et
la détermination de la chaîne de spectres audio d'aide au sommeil selon les un ou plusieurs spectres audio d'aide au sommeil cibles et les générations respectives générées de spectres audio d'aide au sommeil cibles.

5. Procédé selon la revendication 1, dans lequel l'obtention de l'état de sommeil de l'utilisateur comprend:
l'obtention d'une pluralité de paramètres physiologiques de l'utilisateur collectés par un dispositif portable, dans lequel la pluralité de paramètres physiologiques comprend au moins l'un parmi:un nombre de retournements, une fréquence cardiaque, une pression artérielle, une fréquence respiratoire et une fréquence de mouvement de tête; et
la détermination des un ou plusieurs paramètres d'état de sommeil de l'utilisateur selon la pluralité de paramètres physiologiques.

6. Appareil (400) de génération d'un audio d'aide au sommeil, comprenant :
un premier module d'acquisition, configuré pour obtenir une pluralité de spectres audio d'aide au sommeil;
un second module d'acquisition (410), configuré pour traiter la pluralité de spectres audio d'aide au sommeil avec un algorithme génétique, pour obtenir une chaîne de spectres audio d'aide au sommeil; et
un premier module de génération (420), configuré pour générer l'audio d'aide au sommeil selon la chaîne de spectres audio d'aide au sommeil;
dans lequel le premier module de génération (430) comprend:
une première unité d'acquisition, configurée pour obtenir un ou plusieurs paramètres d'état de sommeil d'un utilisateur pendant la lecture de l'audio d'aide au sommeil ;
une première unité de génération, configurée pour déterminer une valeur d'aide au sommeil de l'audio d'aide au sommeil selon les paramètres d'état de sommeil et un réseau d'identification d'aide au sommeil pré-entraîné; et
une seconde unité de génération, configurée pour supprimer, en réponse au fait que la valeur d'aide au sommeil de l'audio d'aide au sommeil est inférieure à un second seuil, un spectre audio d'aide au sommeil à partir duquel l'audio d'aide au sommeil est généré à partir de la pluralité de spectres audio d'aide au sommeil, pour obtenir une pluralité de spectres audio d'aide au sommeil mis à jour.

7. Appareil selon la revendication 6, dans lequel le premier module d'acquisition (410) est configuré pour:
obtenir m spectres audio de référence;
identifier chaque spectre audio de référence avec un modèle d'identification généré par entraînement, pour déterminer une valeur d'aide au sommeil de chaque spectre audio de référence; et
déterminer n spectres audio de référence à partir des m spectres audio de référence en tant que pluralité de spectres audio d'aide au sommeil, dans lequel chacun des n spectres audio de référence correspond à une valeur d'aide au sommeil supérieure à un premier seuil, m est supérieur à n et n est un nombre naturel supérieur à 1.

8. Appareil (400) selon la revendication 7, dans lequel le second module d'acquisition (420) comprend:
une unité de traitement, configurée pour traiter la pluralité de spectres audio d'aide au sommeil avec l'algorithme génétique sur la base des valeurs d'aide au sommeil correspondant à la pluralité de spectres audio d'aide au sommeil, pour obtenir la chaîne de spectres audio d'aide au sommeil.

9. Appareil (400) selon la revendication 8, dans lequel l'unité de traitement est configurée pour:
sélectionner un ou plusieurs spectres audio d'aide au sommeil cibles à traiter parmi la pluralité de spectres audio d'aide au sommeil sur la base des valeurs d'aide au sommeil correspondant à la pluralité de spectres audio d'aide au sommeil;
traiter les un ou plusieurs spectres audio d'aide au sommeil cibles en effectuant au moins l'une d'une opération de croisement et d'une opération de mutation, pour générer une pluralité de spectres audio d'aide au sommeil de première génération;
sélectionner un ou plusieurs spectres audio d'aide au sommeil cibles de première génération parmi la pluralité de spectres audio d'aide au sommeil de première génération sur la base de valeurs d'aide au sommeil correspondant à la pluralité de spectres audio d'aide au sommeil de première génération; et
effectuer, de manière répétée jusqu'à ce qu'un nombre d'opérations effectuées atteigne une valeur prédéfinie, au moins l'une de l'opération de croisement et de l'opération de mutation sur la base des un ou plusieurs spectres audio d'aide au sommeil cibles de première génération, et
déterminer la chaîne de spectres audio d'aide au sommeil selon les un ou plusieurs spectres audio d'aide au sommeil cibles et les générations respectives générées de spectres audio d'aide au sommeil cibles.

10. Appareil (400) selon la revendication 6, dans lequel la première unité d'acquisition est configurée pour:
obtenir une pluralité de paramètres physiologiques de l'utilisateur collectés par un dispositif portable, dans lequel la pluralité de paramètres physiologiques comprend au moins l'un parmi: un nombre de fois de retournement, une fréquence cardiaque, une pression artérielle, une fréquence respiratoire ou une fréquence de mouvement de tête; et
déterminer les un ou plusieurs paramètres d'état de sommeil de l'utilisateur selon la pluralité de paramètres physiologiques.

11. Dispositif électronique, comprenant:
au moins un processeur; et
une mémoire connectée en communication à l'au moins un processeur; dans lequel
la mémoire stocke des instructions qui sont exécutables par l'au moins un processeur, et l'exécution des instructions par l'au moins un processeur amène l'au moins un processeur à effectuer le procédé selon l'une quelconque des revendications 1 à 5.

12. Support de stockage non transitoire lisible par ordinateur, sur lequel sont stockées des instructions informatiques, dans lequel les instructions informatiques sont configurées pour amener un ordinateur à effectuer le procédé selon l'une quelconque des revendications 1 à 5.

13. Produit de programme informatique comprenant un programme informatique, dans lequel, pendant l'exécution du programme informatique par un processeur, le procédé selon l'une quelconque des revendications 1 à 5 est effectué.
